(19) Europäisches Patentamt
European Patent Office
Office européen des brevets

(11) Publication number: **0 074 175**
**A1**

# (12) EUROPEAN PATENT APPLICATION

(21) Application number: 82304051.4

(22) Date of filing: 30.07.82

(51) Int. Cl.³: A 61 C 9/00
A 61 K 6/10

(30) Priority: 24.08.81 US 295343

(43) Date of publication of application:
16.03.83 Bulletin 83/11

(84) Designated Contracting States:
AT BE CH DE FR GB IT LI NL SE

(71) Applicant: SYBRON CORPORATION
1100 Midtown Tower
Rochester, NY 14604(US)

(72) Inventor: Freeman, Frank H.
25541 Crystal Springs Court
Farmington Michigan 48018(US)

(74) Representative: Oliver, Roy Edward et al,
POLLAK,MERCER & TENCH High Holborn House 52-54
High Holborn
London WC1V 6RY(GB)

## (54) Dental apparatus and materials for making oral impression trays in situ.

(57) A dental apparatus for making an oral impression tray in situ comprises a transporter member (2), a tray member and a spacer member (20). The transporter member (2) has an arch-shaped base provided with a handle (14) and includes fracture lines (4) for separating parts of the base, apertures (10) into which the impression material intrudes and raised bite stops (12). The tray member placed on the transporter member (2) is a mass of mouldable polymerizable impression material, desirably comprising a resin vehicle component and a filler component, and the spacer member (20) is a pad of uniform thickness placed on the tray member.

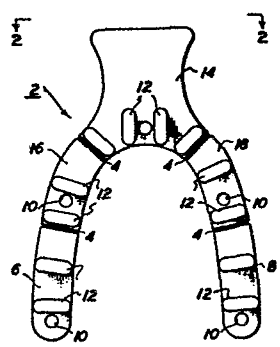


FIG. 1

FIG. 3

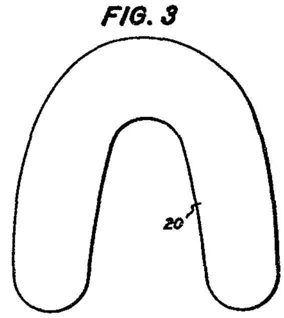


EP 0 074 175 A1

0074175



DENTAL APPARATUS AND MATERIALS FOR MAKING ORAL IMPRESSION TRAYS IN SITU

DESCRIPTION

This invention relates to oral impression trays and is concerned in particular with apparatus and materials for making oral impression trays in situ.

In the art of dentistry, oral impressions are taken by the dentist as a starting point in the fabrication of a variety of restorations and dental appliances. A number of impression material systems are available to meet the needs and varied skills of dentists. The list of available materials includes a variety of elastomeric compositions based upon polysulphides, addition silicones, condensation silicones, polyethers and other elastomers. These materials typically are two-part systems, consisting of a "base" composition and a "catalyst" composition which, when mixed, convert the fluid resin of the base composition into an elastomer. After being properly proportioned and mixed, the materials are conveyed into the oral cavity upon some type of "tray." Dental impression trays usually are re-usable, they are typically of metal or plastics construction and they are designed to follow approximately the shape of either the mandibular or the maxillary arch. They are usually provided in a series of sizes, so as to permit a close fit to the oral

area under consideration.

A similar variety of cheaper and less rigid disposable trays are also available. Again, these only approximately fit the dental arch and, in general, are less structurally strong and rigid. All of these "standard" trays give only an approximate "fit" over the oral area of which an impression is needed. In some areas, the trays may be nearly touching the oral tissues and in other areas a considerable space may exist between the tray and the oral tissues. These variations in spacing result in a correspondingly varying bulk of impression material within the trays. This variation in material bulk contributes to inaccuracies in the oral impressions made and to wastage of these expensive impression materials. Variations in tissue compression and variations in polymerization shrinkage are among other factors which cause inaccurate impressions to be made.

In an effort to control tissue reactions better and to optimize the stresses induced by polymerization shrinkage of the impression material, more discriminating dentists use custom-made trays for taking impressions. This ensures improved impression accuracy and minimizes future problems and remakes. Currently, custom trays are produced by an indirect (extra-oral) technique, as follows:

1. A "snap" impression is taken of the oral structure, using a cheap impression material. The greatest accuracy is neither required nor sought.

2. An artificial stone model is poured into the "snap" impression and allowed to harden for approximately one hour. This model is thus a positive of the oral structure and is used for bench (as distinguished from in situ) construction of an oral impression tray.

3. The tray is made of a plastics material,

usually methyl methacrylate, which cures at room temperature. This involves cutting a suitable "spacer" and placing it over the positive model in the area of which an impression is required. This "spacer" ensures a reasonably uniform space for the final impression material and is removed before using the tray. A handle may also be formed on the tray to facilitate handling and removal of the final impression from the oral cavity. The complete tray, which is thus a spaced negative of the positive model, is removed from the model upon completion of polymerization. It is trimmed, cleaned, separated from the spacer and coated with an adhesive to help retain the final impression material. The tray is now ready for taking the impression.

In contrast to the prior art apparatus and methods summarised above, the present invention provides a "direct oral tray" procedure (intra-oral), which eliminates steps 1 and 2 of the prior art indirect (extra-oral) tray technique. This results in appreciable savings for the dentist in both time and materials.

According to one aspect of the invention, an apparatus, for making an oral impression tray in situ, is characterized by a transporter member for supporting a body of mouldable impression tray material and conveying it into and out of a patient's oral cavity and during subsequent processing of the tray material, the transporter member comprising an arch-shaped flat or curved base and a handle extending from the base, a plurality of fracture lines in the base to facilitate selective removal of pieces of the base, a plurality of apertures in the base for receiving impression tray material and so mechanically locking the tray material together with the base, and a plurality of raised abutments on the base for serving as positive bite stops relative to the base.

A dental apparatus, according to a particularly preferred form of the invention, includes a transporter member, a tray member and a spacer member, wherein:

(a) the transporter member comprises an arch-shaped flat or curved base and a handle extending from the base, a plurality of fracture lines in the base to facilitate selective removal of pieces of the base, a plurality of apertures in the base for receiving mouldable impression tray material and so mechanically locking the material together with the base, and a plurality of raised abutments on the base for serving as positive bite stops relative to the base,

(b) the tray member comprises a mass of mouldable polymerizable material placed on the transporter member and intruded into at least one of the apertures in the base, and

(c) the spacer member comprises a pad of uniform thickness placed on the tray member and serves to form a controlled space between the polymerizable material and the oral structure from which the impression is taken and to prevent the polymerizable material penetrating between the patient's teeth,

whereby the transporter, tray and spacer members form an oral impression tray in situ when assembled and introduced into the patient's oral cavity and the patient bites on to the assembly to the bite stops of the transporter member.

According to another aspect of the invention, a composite material suitable for use as a direct oral impression tray material includes a resin vehicle component comprising an acrylic resin having a molecular weight in the range from 200 to 700 and a filler component selected from silica, alumina, metal oxides, metal salts, silicates, powdered metals, organic plastics materials, insoluble organic materials, resorbable organic materials, resorbable inorganic materials and

and metal organic complexes.

Briefly, the present invention provides a means for making an oral impression tray in situ, preferably using in combination a transporter member, a tray member and a spacer member, such as indicated above. In order that the apparatus of the invention in particular may be clearly and fully understood, reference is made to the accompanying drawings, in which embodiments of the apparatus of the invention are shown, by non-limitative illustration; in the drawings:

Figure 1 shows a plan view of a preferred form of transporter member of this invention;

Figure 2 shows an end view of the transporter member, as seen in the direction indicated by 2-2 of Figure 1;

Figure 3 shows a plan view of a flat spacer pad;

Figure 4 shows an end view of the spacer pad of Figure 3;

Figure 5 shows a plan view of a formed spacer, which is shown also in section in Figure 5a;

Figure 6 shows a side view of an alternative form of the transporter member of Figure 1.

The Transporter Member

Convenience, accuracy and control in producing oral impressions are greatly enhanced if the tray material is carried to the mouth, removed from the mouth and processed on a transporter, such as a transporter member of this invention. The transporter member of the invention, one embodiment of which is shown in Figs. 1 and 2 and another embodiment of which is shown in Fig. 6, is a generally flat or curved surface receptacle, shaped so as to approximate to the human dental arch form and made of plastics material, metal or other suitable material.

Its purpose is to lend convenience, stability and control to the placing of mixed but unset tray-forming material in the oral cavity and removing it after its shaping and polymerization. The transporter member further aids in handling the tray in the subsequent steps of impression taking and model pouring.

Figures 1 and 2 are plan and end views of a typical form of a transporter member according to this invention. Preferably, practice in accordance with this invention involves the use of a disposable transporter member 2, designed so that a single unit, made in one size only, can be cut, broken or trimmed to serve a variety of impression tray-moulding needs. In its entirety, this single unit member provides support for full arch maxillary or mandibular tray impressions. A plurality of fracture lines made in the transporter member, 2, such as the notches and/or score lines 4 shown in Fig. 1, permit the unit 2 to be broken into right or left parts if desired, which can then be used for right or left mandibular or maxillary quadrant impressions. The posterior sections 6,8 of the transporter member 2 can also be selectively removed, the dentist then using only the anterior portions 16,18 to serve as an anterior quadrant impression tray.

A plurality of apertures, such as the holes 10, are provided at various places along the transporter member 2, so that the tray material can be impressed into the holes 10 to provide mechanical locking of the impression tray to the transporter member 2.

A plurality of raised sections or abutments 12, which are typically 1.5 mm (1/16") in thickness, are provided as bite stops, for either maxillary or mandibular tray construction. In use of the transporter member 2 with tray material on it, as described in more detail below, these abutments 12 ensure that a small but adequate thickness of impression tray material is provided over the occlusal plane. Minimizing the thickness of material in this area helps to ensure that an adequate amount of material is available for moulding the tray flanges.

The front end portion 14 is a handle for the transporter member 2 and the materials carried upon it in use.

A re-usable transporter member, as an alternative, offers the advantages of greater strength and rigidity, as compared with disposable plastics material transporter members. Such re-usable transporter members are preferably supplied in at least four configurations:

1. Full arch (maxillary or mandibular);
2. Half arch, right (maxillary or mandibular);
3. Half arch, left (maxillary or mandibular);
4. Anterior arch (maxillary or mandibular).

This multiplicity of forms, with attendant costs and inventory problems, is an economic disadvantage of re-usable units, though such units may provide some cost economies over a period of time, if clean-up time is not charged against them.

<u>The Spacer Member</u>

In the process of producing a direct oral impression tray, two functions must be fulfilled by some type of spacer member. The spacer must (1) cover the interstitial spaces between standing teeth, within the area where the impression is required, so as to prevent entry of the impression material there, and (2) provide a space within the final impression tray for the desired thickness of impression material.

The requirements of a suitable spacer member

are:

1. To adapt easily and well to oral tissues;
2. To be compatible with direct oral tray material;
3. To be insoluble in saliva;
4. To avoid contamination of the surface of the final tray, i.e., so as not to interfere with adhesives applied to the tray surface;
5. To be easily removable from the final impression tray;
6. To be cheap, as spacer members are disposable;
7. To be essentially odourless, tasteless and non-toxic.

Various materials, including rubber waxes, cloth, "Cellophane", plastics films and mouldable clays, etc., have been evaluated for this intended use. The two most acceptable types of materials found to date are preformed foamed plastics materials of suitable density, hardness and thickness and high wet-strength paper of suitable thickness.

To attain the desired "space" for the final impression material within the impression tray, a thickness of approximately 2 mm (1/12") is desired. A single spacer design has been found adequate for both maxillary and mandibular trays for each type of material considered. These configurations are shown in Figures 3, 4 and 5.

Figures 3 and 4 show plan and edge views of an arch-shaped high wet-strength paper or gauze spacer 20 which in use is moistened and placed upon a rope of tray material, which is in turn positioned on the transporter member.

Figures 5 and 5a show a preformed plastics material spacer 30, made of a soft resilient foam. The

spacer 30 can most easily be described as being in the general form of an athletes mouthpiece or gumshield, having a bottom 32 and front and rear walls 34, 36 defining a channel 38.

The functions of the transporter member, such as that shown at 2 in Figs. 1 and 2, and of the spacer member, such as that shown at 20 in Figs. 3 and 4 or that shown at 30 in Figs. 5 and 5a, are best understood in relation to the procedure for making a customized oral impression tray by the direct technique. It must be appreciated that a stepwise description of any technique is only one way of achieving the final objective; some details can be altered without departing from the purpose of the procedure:

1. A properly proportioned mix of the direct oral tray material is prepared according to the directions for the material selected.
2. A "rope" is formed of the mixed tray material and this is laid on the transporter frame or member 2, previously trimmed to cover the area of oral structure of which an impression is required.
3. A suitable "spacer" is then pressed into the plastic, unset tray material, so as to impress the tray material into the locking holes in the transporter frame, such as the holes 10 in the member 2. The basic procedure varies from this point, depending upon the type of spacer member being used:
   (a) With a soft high drape (i.e. wet paper) spacer 20, of the type shown in Figures 3, 4, the assembled transporter member 2, with the rope of tray material and the spacer 20, is carried into the oral cavity and seated over

the oral structure of which an impression is required. Care must be exercised to ensure that the spacer 20 covers all interstitial spaces, so as to prevent intrusion of the tray material between the teeth. The patient is then requested to bite gently until the stops 12 on the transporter frame 2 are contacted. The operator then checks for proper distribution of tray material to form the required tray flanges. The tray is then formed by the operator manipulating the patient's lips and cheeks to form the labial and anterior flanges. The lingual flanges are finger-moulded by the operator, taking care to not disturb the completed labial and anterior flanges. Once the tray form is complete, it is held motionless until polymerization of the tray material advances to a state of strength and hardness to permit removal of the tray. This is accomplished in five to seven minutes from the beginning of mixing of the tray material.

(b) With a preformed semi-rigid spacer 30, of the type shown in Figures 5 and 5a (the preferred type), more of the tray can be formed outside the oral cavity, with somewhat better control over the distribution of material and greater convenience. The basic tray form can be completed by moulding the prepared tray material over the convex side of the spacer 30 before locating the assembly in the oral cavity. The semi-completed tray is then shaped to final form in the mouth. Again, the completely formed tray must then be held

motionless until polymerization proceeds to a point where sufficient hardness and strength are developed for it to be safe to remove the tray. This is accomplished in five to seven minutes from the start of mixing of the tray material.

The transporter member 2 provides a rigid base to carry the unset tray material conveniently into the mouth. It provides a means for controlling the thickness of the tray over the occlusal surfaces and it provides a convenient handle for removing the polymerized tray from the oral cavity. The handle also aids the operator to hold the tray during the subsequent operations of washing, trimming, removing the spacer, painting on the adhesive, filling the tray with impression material and, again, carrying the tray into the oral cavity to take the impression. Removal of the impression, cleaning and pouring of the model are all facilitated by the transporter handle 14.

Figure 6 shows an alternative form of transporter member 2 which is generally shaped or curved to correspond to the oral cavity.

<u>Method</u>

The above description of various forms of the apparatus of the invention and the use of such forms of apparatus thus exemplifies the general direct oral impression tray technique of this invention which is carried out as follows:

1. Appropriate quantities of a two-part material system are mixed together.

2. The completed mix is then formed into a rope and placed upon a "transporter" frame.

3. A previously-prepared "spacer" is then placed upon the rope of mixed material, sufficient pressure being applied to ensure adhesion of the spacer to the tray material and of the tray material to the

transporter frame. The tray material should be pressed sufficiently so as to intrude into locking holes provided in the transporter frame.

4. The assembly is carried to the mouth and centered over the area of interest. Biting pressure by the patient then forms the mass of tray material to the correct thickness against stops on the transporter frame.

5. The operator then quickly finger-moulds the exuded tray material to form the desired lingual tray flanges and shape. Muscle and cheek trimming are effected as in taking a normal impression to form the anterial and labial tray flanges. Within five to seven minutes after starting to form the mix, a completed customized impression tray is removed from the mouth.

6. The assembly is washed and trimmed and the spacer is then removed from the tray. This leaves a controlled space for the impression material used in the next step of the impression taking procedure. The spacer not only produces the correct space for the impression material, but also prevents the tray material from penetrating between standing teeth, thus ensuring easy removal of the polymerized impression tray from the mouth.

The elimination of a "snap" impression and of the pouring of a stone model, as in the prior art method described, makes a major saving of both time and material for the dentist. The direct oral tray procedure may also permit the dentist to complete the taking of an impression in a single patient appointment period, thus improving operating efficiency and eliminating the inconvenience of a second appointment for the patient.

Materials

The new tray composition comprising the composite material of the invention has the following characteristics, which make it particularly desirable as a direct oral tray material:

1. It is easily and quickly prepared at the time it is needed;
2. It is cohesive and plastic without being objectionably adhesive to the hands;
3. It is essentially odourless and tasteless;
4. It does not produce tissue burn, sting or sensation;
5. It polymerizes with very little exothermal heat or temperature rise;
6. It has low polymerization shrinkage;
7. It has adequate physical strength and toughness for taking oral impressions;
8. It leaves the mouth clean and free of residue;
9. It has an acceptable colour or can be coloured so as to facilitate location and moulding within the oral cavity;
10. The monomer or monomers are non-volatile and non-invasive to the oral tissues.

Dental practice with the apparatus and in accordance with the method of this invention preferably involves the use of mono-, di-, tri- or tetramethacrylate monomers with molecular weights in the range from 200 to 700. In general, resins or blends of resins within this molecular weight range have an exothermic reaction of sufficiently low intensity (as compared to prior art monomers such as methyl methacrylate) to be useful in compositions which are intended to be polymerized in situ, against human hard or soft tissues, without causing thermal damage to such tissues.

The volatility properties, viscosity characteristics and the potential biological tolerance of these resins are generally more favourable than those of lower molecular weight monomers. The polymerization of methyl methacrylate is an exothermic reaction which would burn the tissue if carried out in situ. Either aromatic or aliphatic mono-, di-, tri- or tetra-functional methacrylate monomers within the molecular weight range from 200 to 700 may be useful as single monomers or as blends of monomers for specific applications.

Depending upon the product characteristics desired, various fillers may be used. Silicas, aluminas, clays, powdered metals, metal oxides, metal silicates, metal salts, plastics materials, organic materials and many others may be used to impart specific product characteristics, i.e., radiopacity, radiolucency, etc., electrical conductivity, control of magnetic fields, soluble ion release and other characteristics.

The filler or fillers can be utilized in a broad range of particle size distributions, from submicron to very coarse, singly or in blends, and with or without surface treatments to influence wetting, to promote chemical bonding with the resin matrix or other special characteristics, as specific uses require. A small amount of a suitable wetting agent is desirably used to facilitate wetting and dispersion of the filler particles in the resin or blended resin vehicle.

The material system is suitable for a variety of dental or medically oriented uses where the material has to be polymerized in situ against or in close proximity to living tissues. A number of non-invasive applications can be suggested. These include, but are not limited to:

1. A direct chairside hard denture reline;

2. Oral splints;

3. Periodontal packs;

4. Fingernail overlay;

5. Electrical and/or magnetic shielding devices;

6. Electrical and/or magnetic focusing devices.

Products formulated within the concept of this invention may be supplied as two-part products, i.e., powder and liquid components, two-paste components, paste and liquid components, etc. A skilled formulator can convert the system to any desired one of these product types.

Example 1

A specific example of the simplest product type, namely a powder and liquid component direct oral tray material, can be used to illustrate the concept of the invention:

| | |
|---|---|
| (a) Resin vehicle/binder (liquid) | |
| Ethoxylated bis-phenol-A-di-methacrylate | 98.85% |
| Wetting agent (oleoyl sarcosine) | 1.00% |
| 2,2$^{1}$-p-Tolylimino-diethanol* | 0.15% |
| (b) Filler (powder) | |
| Calcium carbonate, ground, average particle size 7µ (7 x $10^{-3}$ mm) | 99.55% |
| Benzoyl peroxide* | 0.45% |

* subject to adjustment, as required

Mixes comprising four to five parts of the powder with one part of the liquid, when spatulated to a cohesive mass and then rolled between the palms for 30 seconds, e.g. so as to form a "rope", produce a cohesive plastic mouldable mass, which has excellent handling characteristics and properties, for manipulating and forming a direct oral impression tray within the oral cavity. The mix working time or period of

acceptable mouldability can be adjusted by changes in the levels of benzoyl peroxide in the powder or the 2,2[1]-p-tolylimino-diethanol in the liquid. Normally, a working time of three to four minutes is adequate. Overall, the oral impression tray device can be removed from the mouth within five to seven minutes from the start of formation of the mix.

Example 2

A second example of a direct oral tray material, formulated as a two-paste product, is as follows:

Paste B (Base Paste) and Paste C (Catalyst Paste) are essentially the same composition, except that the "B" paste contains an amine promoter and a colouring agent, if one is desired, while the "C" paste contains the benzoyl peroxide as a free radical polymerization initiator and preferably no colouring agent. However, the pastes may differ in composition and characteristics, if such differences are advantageous or needed.

Paste "B", the base, contains a small quantity of a suitable amine, as an initiator for the free radical polymerization system, which may be selected from a variety of amines. The final choice and level of amine content will depend upon product requirements, individual preference, cost and the rate of polymerization desired. Aniline, dimethyl-p-toluidine, 2,2[1]-m-tolylimino-diethanol, 2,2[1]-p-tolylimino-diethanol or other amines may be found suitable. The selection of a colouring agent will depend upon product requirements, compatibility in the formulation, toxicity restrictions and colour characteristics.

Paste "C", the catalyst paste, contains a small amount of benzoyl peroxide. It is the decomposition of the benzoyl peroxide by the amine in the base paste which produces the free radicals which, in turn, initiate

the polymerization of the paste vehicle monomers. Other peroxides may be substituted, but benzoyl peroxide is currently preferred.

Two-Paste Preferred Compositions:

Paste "B", Base

| | |
|---|---|
| Ethoxylated bisphenol-A-dimethacrylate | 12.40% |
| BisGMA (2,2-bis)p-(2-hydroxy-3[1]-methacryloxypropoxy-phenyl)-propane | 3.00% |
| Wetting agent (i.e., oleoyl sarcosine) | 0.10% |
| 2,2[1]-p-tolylimino-diethanol | 0.15% |
| Super Flex 200 (Pfizer Minerals, Pigments and Metals Division) | 13.00% |
| Camel Carb (Flintkote Stone Products Co.) | 71.35% |
| FDA Approved Colour - | None or as required |

Paste "C" Catalyst

| | |
|---|---|
| Ethoxylated bisphenol-A-dimethacrylate | 12.40% |
| BisGMA | 3.00% |
| Wetting agent (i.e., oleoyl sarcosine) | 0.10% |
| Benzoyl peroxide | 0.10% |
| Super Flex 200 | 13.00% |
| Camel Carb | 71.40% |

Two resins have been selected which are blended together in one product to serve as both paste vehicle and the polymerizable binder. These resins are:

I. Ethoxylated Bisphenol A Dimethacrylate

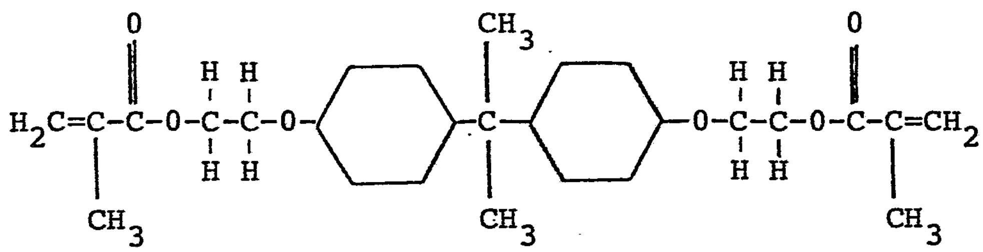

MW = 452 Viscosity at 25°C = 1600 cps

and

II. BisGMA /(2,2-bis)-p-(2-hydroxy-3[1]-methacryloxy-propoxy-phenyl)-propane/

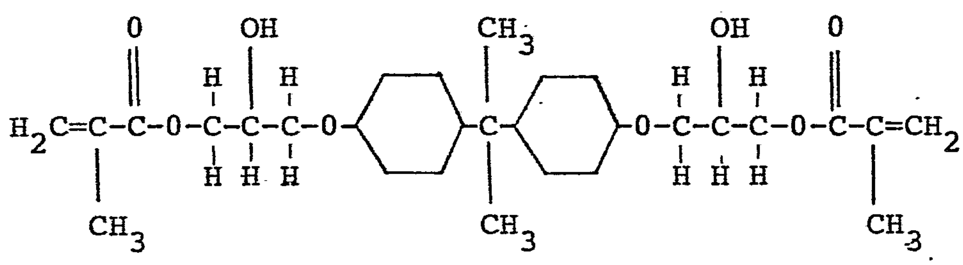

MW = 512 Viscosity at 25°C = 1,500,000 cps

Neither resin alone has given acceptable two-paste product handling characteristics. By selectively blending the resins in the approximate ratio of four parts of I to one part of II, a paste vehicle is obtained which gives to the formulated paste the desired balance between cohesion and adhesion properties. While these two resins are the preferred combination for this particular product, the concept covers other resin combinations which serve equally well, or even better, for other product characteristics. For example, the lower viscosity resin (I) can be replaced, in some proportion, by tri-ethyleneglycol dimethacrylate, MW 286, trimethylolpropane tri-methacrylate, MW 338, polyethylene-glycol di-methacrylate, MW 330, 1,3-butylene-glycol di-methacrylate, MW 226, diethylene-glycol di-methacrylate, MW 330, and propoxylated bis-phenol-A-di-methacrylate, as examples of possible substitutions. Each pair of monomers and each variation of monomer ratio will have its own characteristics, which may be preferred for a specific application. Likewise, the higher viscosity resin (II) may be replaced by di-acetyl-BisGMA resin, di-benzoyl-substituted BisGMA resin or urethane-substituted BisGMA resin or other compatible high viscosity resin within the MW range of 200-700, and diluted with any of the lower viscosity resins (I) and its substituted monomers.

CLAIMS:

1. A dental apparatus for making an oral impression tray in situ,
characterized by
a transporter member for supporting a body of mouldable impression tray material and conveying it into and out of a patient's oral cavity and during subsequent processing of the tray material, the transporter member comprising an arch-shaped flat or curved base and a handle extending from the base, a plurality of fracture lines in the base to facilitate selective removal of pieces of the base, a plurality of apertures in the base for receiving impression tray material and so mechanically locking the tray material together with the base, and a plurality of raised abutments on the base for serving as positive bite stops relative to the base.

2. A dental apparatus for making an oral impression tray in situ,
characterized in
that the apparatus includes a transporter member, a tray member and a spacer member, wherein:

(a) the transporter member comprises an arch-shaped flat or curved base and a handle extending from the base, a plurality of fracture lines in the base to facilitate selective removal of pieces of the base, a plurality of apertures in the base for receiving mouldable impression tray material and so mechanically locking the material together with the base, and a plurality of raised abutments on the base for serving as positive bite stops relative to the base,

(b) the tray member comprises a mass of mouldable polymerizable material placed on the transporter member and intruded into

at least one of the apertures in the base, and

(c) the spacer member comprises a pad of uniform thickness placed on the tray member and serves to form a controlled space between the polymerizable material and the oral structure from which the impression is taken and to prevent the polymerizable material penetrating the patient's teeth,

whereby the transporter, tray and spacer members form an oral impression tray in situ when assembled and introduced into the patient's oral cavity and the patient bites on to the assembly to the bite stops of the transporter member.

3. A polymerizable biologically-accepted composite material for use as a direct oral impression tray material,

characterized by

a resin vehicle component comprising an acrylic resin having a molecular weight in the range from 200 to 700 and a filler component selected from silica, alumina, metal oxides, metal salts, silicates, powdered metals, organic plastics materials, insoluble organic materials, resorbable organic materials, resorbable inorganic materials and metal organic complexes.

4. A direct oral impression tray material comprising a resin vehicle component and a filler component,

characterized in

that the resin vehicle component comprises an acrylic resin having a molecular weight in the range from 200 to 700 and the filler component comprises plain or surface-treated calcium carbonate.

5. A material according to claim 4, in which the acrylic resin comprises ethoxylated bis-phenol-A di-methacrylate and the calcium carbonate filler has an average particle size of 7 x $10^{-3}$mm.

6. A material according to claim 4 or 5, in which the resin vehicle component further comprises a wetting agent and a polymerization initiator and the filler component includes a polymerization initiator comprising benzoyl peroxide.

7. A material according to claim 6, in which the polymerization initiator comprises $2,2^{1}$-p-tolyl-imino-diethanol or $2,2^{1}$-m-tolylimino-diethanol.

8. A material according to claim 6 or 7, in which the wetting agent is oleoyl sarcosine.

0074175

1/2

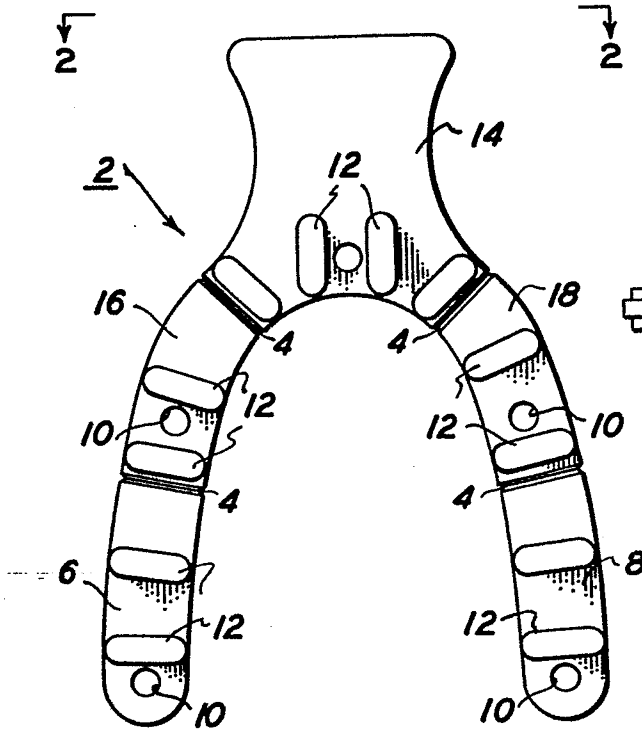


FIG. 1

FIG. 2

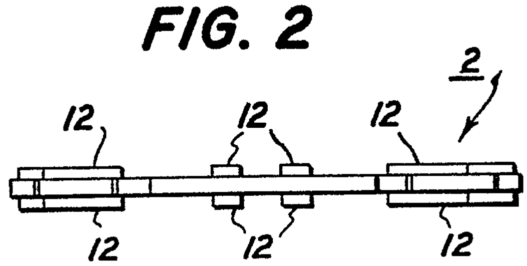


FIG. 3

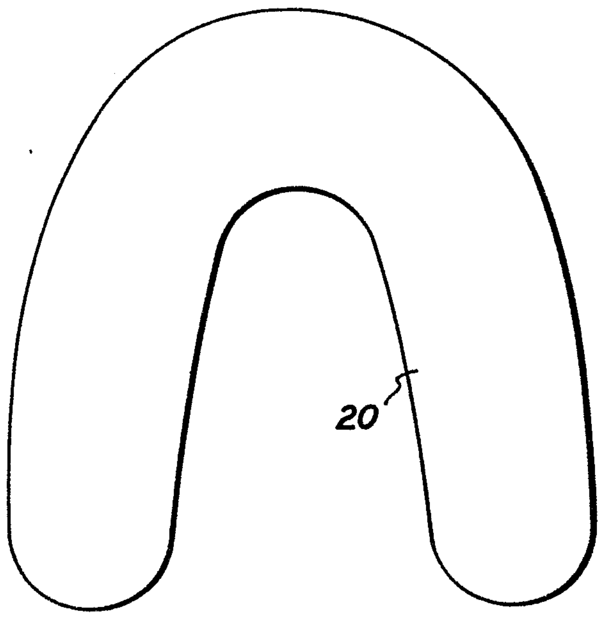


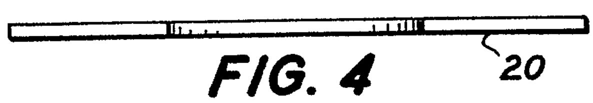


FIG. 4

FIG. 5

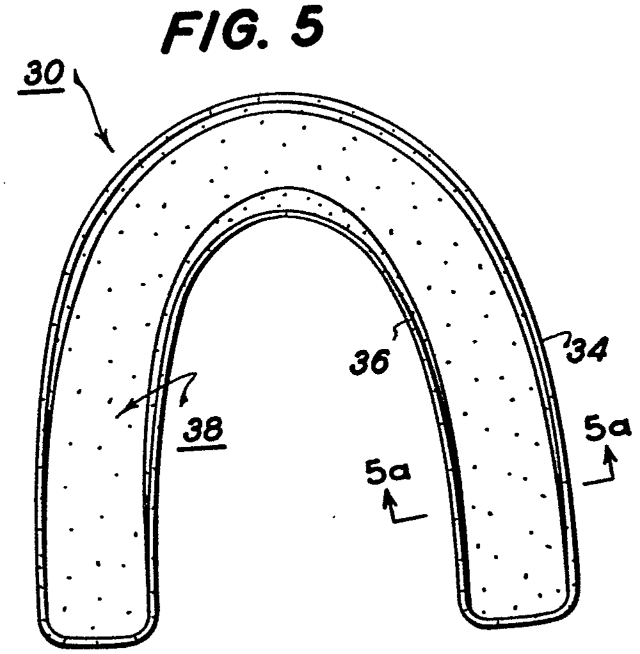


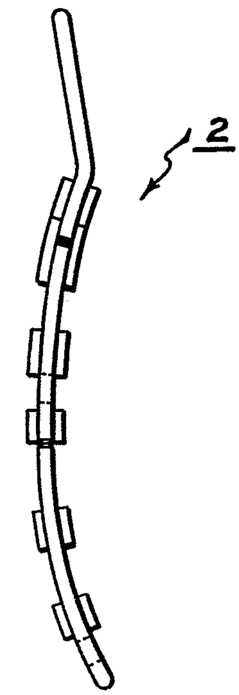


FIG. 6

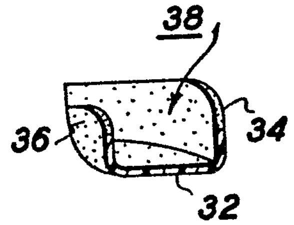


FIG. 5a

European Patent Office

**EUROPEAN SEARCH REPORT**

0074175

Application number

EP 82 30 4051.4

## DOCUMENTS CONSIDERED TO BE RELEVANT

| Category | Citation of document with indication, where appropriate, of relevant passages | Relevant to claim |
|---|---|---|
| Y | US - A - 3 390 457 (ZATZ)<br>* fig. 8 *<br>-- | 1,2 |
| Y | US -A - 3 056 205 (ENNOR)<br>* fig. 1 *<br>-- | 1,2 |
| Y | US - A - 2 634 500 (McADOO)<br>* column 4, lines 42 to 47 *<br>-- | 1 |
| Y | US - A - 2 963 786 (BROWNING)<br>* column 1, lines 31 to 49 *<br>-- | 1 |
| Y | US - A - 3 537 179 (PARKER et al.)<br>* column 2, lines 53 to 66 ; fig. 1, 3 *<br>-- | 1 |
| Y | US - A - 1 763 553 (DENNIS)<br>* page 1, lines 49 to 97 *<br>-- | 2 |
| Y | DE - A1 - 2 512 443 (BISICO LUDWIGS & MARTEN OHG)<br>* complete document *<br>-- | 2 |
| A | DE - U - 1 681 690 (BREMER GOLDSCHLÄGEREI W. HERBST)<br>* page 2, lines 6 to 14 *<br>-- | 1 |
| | .../... | |

**CLASSIFICATION OF THE APPLICATION (Int. Cl. 3)**

A 61 C 9/00
A 61 K 6/10

**TECHNICAL FIELDS SEARCHED (Int.Cl. 3)**

A 61 C 9/00

**CATEGORY OF CITED DOCUMENTS**

- X: particularly relevant if taken alone
- Y: particularly relevant if combined with another document of the same category
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document

The present search report has been drawn up for all claims

| Place of search | Date of completion of the search | Examiner |
|---|---|---|
| Berlin | 16-11-1982 | SIMON |

European Patent Office

# EUROPEAN SEARCH REPORT



## DOCUMENTS CONSIDERED TO BE RELEVANT

| Category | Citation of document with indication, where appropriate, of relevant passages | Relevant to claim |
|---|---|---|
| A | DD - A - 135 995 (ESPE FABRIK PHARMAZEUTISCHER PRÄPARATE GMBH)<br>* claim 3 *<br>-- | 3-5 |
| A | US - A - 2 745 813 (LOGEMANN et al.)<br>* column 1, line 18 to column 2, line 6 *<br>-- | 3,4 |
| A | DE - A - 136 923 (TUREAUD)<br>* page 11, lines 14 to 26 *<br>-- | 3,6 |
| A | DE - C - 1 044 351 (FARBENFABRIKEN BAYER)<br>-- | |
| A | DE - A - 2 057 978 (FARBWERKE HOECHST)<br>-- | |
| A | Chemical Abstracts vol. 75, no. 8, 23 August 1971,<br>Columbus, Ohio, USA<br>E.J. MOLNAR et al. "Plastic impression compositions"<br>column 1, abstract no. 50144u<br>no. 50144u<br>-- | |

CLASSIFICATION OF THE APPLICATION (Int. Cl.³)

TECHNICAL FIELDS SEARCHED (Int. Cl.³)

.../...

European Patent Office

**EUROPEAN SEARCH REPORT**

0074175
Application number

EP 82 30 4051.4



| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| A | Chemical Abstracts vol. 78, no. 4, 29 January 1973, Columbus, Ohio, USA E.J. MOLNAR et al. " Ear mold and dental impression plastics" column 1, abstract no. 17243q<br>-- | | |
| A | Chemical Abstracts vol. 84, February 1976, Columbus, Ohio, USA T. SHIOTSU "Photopolymerizable compositions for dental uses" column 1, abstract no. 35364 h<br>-- | | TECHNICAL FIELDS SEARCHED (Int. Cl.³) |
| A | Chemical Abstracts vol. 85, no. 14, 4 October 1976, Columbus, Ohio, USA R. MICHL et al. "Dental materials" page 99, column 1, abstract no. 99224r<br>-- | | |
| | .../... | | |

0074175

European Patent Office

# EUROPEAN SEARCH REPORT

Application number

EP 82 30 4051.4

- page 4 -

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | Chemical Abstracts vol. 87, no. 24, 12 December 1977<br>Columbus, Ohio, USA<br>T. HIRASAWA et al. "Tray resins"<br>column 2, abstract no. 189399x<br>& J. Dent. Eng. no. 40, 1977, pages 20 to 29<br>---- | | |
| | | | TECHNICAL FIELDS SEARCHED (Int. Cl.3) |

EPO Form 1503.2 06.78